# EUROPEAN PATENT SPECIFICATION

(11) **EP 3 883 474 B1**
(45) Date of publication and mention of the grant of the patent: **27.04.2022**
(21) Application number: 19798100.4
(22) Date of filing: 11.11.2019
(51) Int. Cl.: A61B 6/03, A61B 6/00

(54) **APPARATUS FOR ALVEOLI BASED VISUALIZATION IN DARK-FIELD OR PHASE-CONTRAST X-RAY IMAGING**
VORRICHTUNG ZUR ALVEOLENBASIERTEN VISUALISIERUNG IN DER DUNKELFELD- ODER PHASENKONTRASTRÖNTGENBILDGEBUNG
APPAREIL DE VISUALISATION À BASE D'ALVÉOLES EN IMAGERIE PAR RAYONS X À CHAMP SOMBRE OU À CONTRASTE DE PHASE

(30) Priority: 20.11.2018 EP 18207230
(43) Date of publication of application: 29.09.2021
(73) Proprietor: Koninklijke Philips N.V., 5656 AG Eindhoven (NL)
(72) Inventor: YAROSHENKO, Andre, 5656 AE Eindhoven (NL); KOEHLER, Thomas, 5656 AE Eindhoven (NL); MAACK, Hanns-Ingo, 5656 AE Eindhoven (NL)
(74) Representative: Philips Intellectual Property & Standards
(86) International application number: PCT/EP2019/080776
(87) International publication number: WO 2020/104224

(56) References cited:
- S. SCHLEEDE ET AL: "Emphysema diagnosis using X-ray dark-field imaging at a laser-driven compact synchrotron light source", PROCEEDINGS OF THE NATIONAL ACADEMY OF SCIENCES OF THE UNITED STATES OF AMERICA, vol. 109, no. 44, 16 October 2012 (2012-10-16), pages 17880-17885, XP055260851, US ISSN: 0027-8424, DOI: 10.1073/pnas.1206684109
- LUKAS B. GROMANN ET AL: "In-vivo X-ray Dark-Field Chest Radiography of a Pig", SCIENTIFIC REPORTS, vol. 7, no. 1, 6 July 2017 (2017-07-06), XP055405084, DOI: 10.1038/s41598-017-05101-w
- MARCUS J KITCHEN ET AL: "X-ray specks: low doseimaging of lung structure and function", PHYSICS IN MEDICINE AND BIOLOGY, INSTITUTE OF PHYSICS PUBLISHING, BRISTOL GB, vol. 60, no. 18, 8 September 2015 (2015-09-08), pages 7259-7276, XP020288165, ISSN: 0031-9155, DOI: 10.1088/0031-9155/60/18/7259 [retrieved on 2015-09-08]
- JANNE VIGNERO ET AL: "Translation from murine to human lung imaging using x-ray dark field radiography: A simulation study", PLOS ONE, vol. 13, no. 10, 29 October 2018 (2018-10-29), page e0206302, XP055592248, DOI: 10.1371/journal.pone.0206302

## Description

### FIELD OF THE INVENTION

The present invention relates to an apparatus for alveoli based visualization in dark-field or phase contrast X-ray imaging, to a system for alveoli based visualization in dark-field or phase contrast X-ray imaging, to a method for alveoli based visualization in dark-field or phase contrast X-ray imaging as well as to a computer program element and a computer readable medium.

### BACKGROUND OF THE INVENTION

Differential phase contrast and dark-field imaging (DPCI and DFI) are promising technologies that will likely enhance the diagnostic quality of X-ray Computer Tomography (CT) and radiography systems. In the past, X-ray dark-field and phase-contrast imaging has demonstrated in several preclinical studies a high potential for an improved diagnosis of pulmonary disorders. Thereby, X-ray dark-field imaging, for example, quantifies the small-angle scattering that is occurring in the object due to differences in the refractive index of different materials. The acquisition of X-ray dark-field and phase-contrast imaging relies on a three grating interferometer, that is used to differentiate between the attenuation of radiation, small-angle scattering and refraction. Thus, imaging with an interferometer provides three independent images: conventional attenuation, dark-field and phase-contrast. The three imaging modalities are intrinsically perfectly registered. The information provided by X-ray dark-field and phase-contrast can be used for diagnostic purposes.

For pulmonary imaging especially X-ray dark-field imaging, but also phase-contrast imaging, has been identified as a possibility to significantly increase the diagnostic sensitivity and specificity. For example, it has been shown that pulmonary emphysema can be significantly better depicted on X-ray dark-field images. For an emphysematous lung, where a lot of alveoli is destroyed, a clear signal decay can be observed in the dark-field signal.

S. Schleede at al, "Emphysema diagnosis using X-ray dark-field imaging at a laser-driven compact synchrotron light source", PNAS, vol. 109, no. 44, 17880-17885, (2012), describes that in the early stages various pulmonary diseases, such as emphysema and fibrosis, the change in x-ray attenuation is not detectable with absorption based radiography. To monitor the morphological changes that the alveoli network undergoes in the progression of these diseases, the paper describes using the dark-field signal, which is related to small angle scattering in the sample. It is described that combined with the absorption based image, the dark-field signal enables better discrimination between healthy and emphysematous lung tissue in a mouse model. All measurements were performed at 36keV using a monochromatic laser driven miniature synchrotron x-ray source (compact light source). The paper presents grating-based dark-field images of emphysematous versus healthy lung tissue, where it is described that the strong dependence of the dark-field signal on mean alveoli size leads to improved diagnosis of emphysema in lung radiographs.

One of the problems that has to be resolved before X-ray dark-field and phase-contrast imaging can be brought into the clinical routine is the appropriate visualization of the dark-field or phase contrast signal to the clinicians. Radiologists have no experience with either of these new imaging modalities. Taking dark-field imaging as an example, this new imaging modality and the quantity of small-angle scattering is not heuristic and will require a significant amount of time to be adopted. Furthermore, one of the promises that is connected with X-ray dark-field imaging is the possibility to quantify and stage disorders like emphysema on radiographic images. However, quantification is not straightforward, due to inter-patient variability.

There is a need to address these issues.

### SUMMARY OF THE INVENTION

It would be advantageous to have improved means of processing dark-field or phase-contrast images in order that interpretation by clinicians is facilitated.

The object of the present invention is solved with the subject matter of the independent claims, wherein further embodiments are incorporated in the dependent claims. It should be noted that the following described aspects and examples of the invention apply also to the apparatus for alveoli based visualization in dark-field or phase-contrast imaging, the system for alveoli based visualization in dark-field or phase-contrast imaging, the method for alveoli based visualization in dark-field or phase contrast imaging, as well as for the computer program element and computer readable medium.

According to a first aspect, there is provided an apparatus for alveoli based visualization in dark-field or phase-contrast X-ray imaging, comprising:
- an input unit;
- a processing unit; and
- an output unit.

The input unit is configured to provide the processing unit with a dark-field or phase-contrast X-ray image of a region of interest of an object. Image data in the dark-field or phase contrast X-ray image are represented in units of a dark-field signal or a phase-contrast signal. The input unit is configured also to provide the processing unit with a calibration relating dark-field signal or phase-contrast signal to an alveoli index in a lung. The processing unit is configured to utilize the calibration to convert the dark-field X-ray image or phase-contrast X-ray image of the region of interest of the object into an alveoli based image of the region of interest of the object, wherein the image data are represented in units of the alveoli index. The output unit is configured to output the alveoli based image.

In other words, the dark-field signal or phase-contrast signal can be normalized to an alveoli index (such as number of alveoli) in order to display the new imaging modalities of dark field or phase contrast data in the alveoli units. This information can be displayed both on a stand-alone image dark-field image or phase-contrast image but also as a (colormap) overlaying a conventional transmission image. The number of alveoli is an easy to understand unit that medical practitioners are familiar with, and enables such medical practitioners to better and easier understand and interpret the image data they are presented with. This will help accelerate the adoption of these new imaging modalities. Furthermore, this heuristic quantity makes quantitative comparison between patients significantly easier.

In this manner, the clinician can interpret the dark-field image or phase-contrast image, to determine for example the number of healthy alveoli on the basis of only a dark-field image or phase-contrast image, without the need for an additional CT scan for that patient, thereby leading to reduced X-ray dosages. The clinician can then decide on the basis of the dark-field image or phase-contrast image that surgical intervention is required, and then a CT scan could be performed in order to evaluate a strategy for surgical intervention, and therefore X-ray dosages are reduced and are only increased when absolutely necessary.

To put this another way, converting the dark-field image or phase contrast image into one represented in the "Alveoli Index" provides a far more intuitive presentation, that helps radiologists detect abnormalities in the lung. The radiologist can detect the pathologies directly from this "alveoli index". By using a calibration to convert the image in this manner, mistakes that could occur through not fully understanding the nature of dark-field, i.e. small angle scattering, is prevented. For example, it is known that if an image is performed in exhalation, then more small-angle scattering is observed than in inhalation, because the alveoli are more condensed, i.e. there are more alveoli in each pixel. While it is not so straightforward to understand this if this is observed in terms of small-angle scattering. However, for a clinician it is quite to interpret the image when converted into the alveoli index, because the clinician can understand that in exhalation there are more alveoli in each pixel. Moreover, the alveoli index provides a number/index that can be used for quantification more easily/intuitively. That is to say, for example it can be established that for a healthy adult in inhalation, the mean alveoli index is not lower than 10000 (this is just an exemplar number). And if the image reveals that the number is lower, then the clinician can immediately appreciate that there is an abnormality in terms of an alveoli index, that means something to them, and the abnormality can thus be detected and perhaps even staged. Thus, doctors are provided with an intuitive number, rather than them having to view and discuss imagery in terms of X-ray small-angle scattering.

The dark-field signal (or phase-contrast signal) can be normalized to the number of alveoli, since the logarithm of the dark-field signal scales linearly with the number of alveoli in the lung and the standard deviation (noise) in the phase-contrast signal scales linearly with the number of alveoli in the lung, and this has been used in determining a calibration that transforms imagery into an understandable for medical practitioners.

The calibration can be generated on one system, and then applied to each subsequent system. Or, the calibration can be generated for each system. But the calibration is not carried out for each patient, but just once in order to determine how much dark-field signal or phase-contrast signal is associated with alveoli.

However, one thing that may have to be taken into account during utilizing of the calibration is the position of the patient, i.e. if the patient is not correctly positioned there could be a mismatch. If the patient is not correctly positioned, but his/her position are known, this can be taken into account for a correct calibration. Therefore, when the calibration is calculated through simulation or through experiment, this can be determined for a number of different positions of the body of the subject. Then, there are calibrations for several different possible positions of the patient, and then interpolation between values for these different positions can be used in determining the calibration from a known positon of the patient now being examined via dark-field or phase-contrast imaging only.

In an example, the calibration relates to how much dark-field signal or phase-contrast signal is associated with an alveolus.

In an example, the calibration is a simulation generated calibration.

In an example, the calibration was generated on the basis of a simulation of at least one sphere having a diameter equivalent to a diameter of a typical alveolus.

In other words, for the simulation, a phantom is used, that has a high resemblance to the alveolus. This can be achieved by simulating a sphere with the same diameter as a typical alveolus and the material strength and properties similar to the tissue properties.

In an example, the calibration is an experimentally generated calibration.

In an example, the calibration was generated on the basis of a comparison between a calibration CT image of a lung and a calibration dark-field image or calibration phase-contrast image of the lung.

CT here means X-ray Computer Tomography.

In an example, the generation of the calibration comprises determining a dark-field signal or phase-contrast signal at one or more positions of the calibration dark-field image or calibration phase-contrast image and determining a number of alveoli corresponding to the one or more positions of the calibration CT image.

In other words, for the experimentally determined calibration dark-field images or phase-contrast images of a lung or parts of a lung are acquired, for example of a healthy patient. Subsequently a high resolution CT scan (resolution has to be high enough to reveal the number of alveoli in the lung) of the same lungs are acquired. Registration of the CT scans to the dark-field radiography images makes it possible to directly count the number of alveoli that caused the dark-field signal or phase-contrast signal.

In an example, determination of the calibration comprises dividing the dark-field signal or phase-contrast signal at the one or more positions of the calibration dark-field image or calibration phase-contrast image by the number of alveoli corresponding to the one or more positions of the calibration CT image.

Thus, the dark-field signal or phase-contrast signal is measured on a healthy patient or for example at least a part of an extracted lung. Then, a high resolution CT is performed, so that the alveoli are visualized and then the number of alveoli in the beam are counted. Registration of the CT/DAX scans can performed as part of this process.

In an example, the region of interest of the object comprises a lung.

In an example, dark-field signal or phase-contrast signal outside of the lung in the dark-field or phase-contrast X-ray image of a region of interest of the object is set to a value that is outside of the range of values in the lung

In an example, the value is set to zero.

In this manner, effects such as e.g. beam hardening that can lead to artificial dark-field signal outside the lung are mitigated. This is because the visualization based on the alveoli index (such as number of alveoli) is performed only for the masked lung, where all the dark-field signal or standard deviation in phase contrast for the noise in the phase-contrast signal outside the lung is set to a uniform value that is different to that in the lung (e.g. zero), in order not to confuse the clinicians.

According to a second aspect, there is provided a system for alveoli based visualization in dark-field or phase-contrast X-ray imaging, the system comprising:
- at least one image acquisition unit; and
- an apparatus for alveoli based visualization in dark-field or phase-contrast X-ray imaging according to the first aspect.

The at least one image acquisition unit is configured to provide the X-ray attenuation image, and to provide the dark-field or phase-contrast X-ray image.

According to a third aspect, there is provided a method for alveoli based visualization in dark-field or phase-contrast X-ray imaging, comprising:
a) providing a dark-field or phase-contrast X-ray image of a region of interest of an object, wherein image data are represented in units of a dark-field or a phase-contrast signal;
b) providing a calibration relating dark-field or phase-contrast signal to an alveoli index in a lung;
c) utilizing the calibration to convert the dark-field or phase-contrast X-ray image of the region of interest of the object into an alveoli based image of the region of interest of the object, wherein the image data are represented in units of the alveoli index; and
d) outputting the alveoli based image.

According to another aspect, there is provided a computer program element controlling apparatus as previously described which, if the computer program element is executed by a processing unit, is adapted to perform the method steps as previously described.

According to another aspect, there is provided a computer readable medium having stored computer element as previously described.

The computer program element, can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for alveoli based visualization in dark-field or phase-contrast X-ray imaging;
Fig. 2 shows a schematic set up of an example of an apparatus for alveoli based visualization in dark-field or phase-contrast X-ray imaging;
Fig. 3 shows a shows a method for alveoli based visualization in dark-field or phase-contrast X-ray imaging;
Fig. 4 shows an example of an X-ray dark-field image represented in an alveoli index of number of alveoli, showing signal strength in alveoli number; and
Fig. 5 shows a schematic set up of an example of a phase-contrast and/or dark-field imaging system.

### DETAILED DESCRIPTION OF EMBODIMENTS

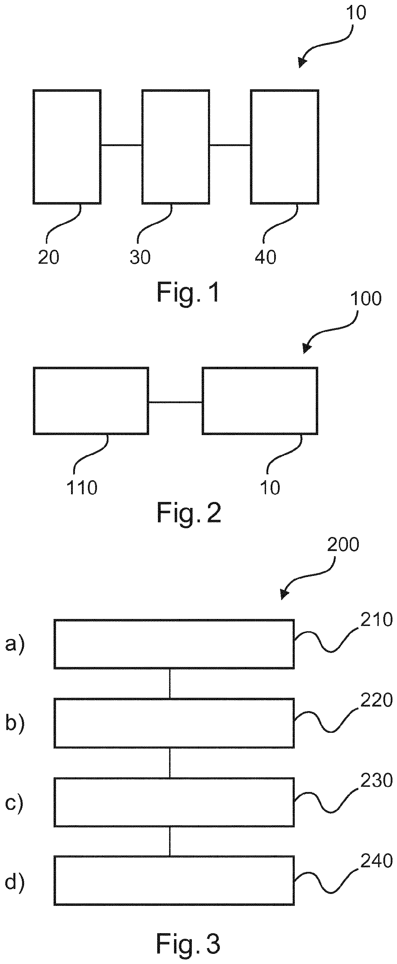

Fig. 1 shows an example of an apparatus 10 for alveoli based visualization in dark-field or phase-contrast X-ray imaging. The apparatus 10 comprises an input unit 20, a processing unit 30, and an output unit 40. The input unit is configured to provide the processing unit with a dark-field or phase-contrast X-ray image of a region of interest of an object. Image data in the dark-field or phase-contrast X-ray image of the region of interest of the object are represented in units of a dark-field signal or a phase-contrast signal. The input unit is configured to provide the processing unit with a calibration relating dark-field signal or phase-contrast signal to an alveoli index in a lung. The processing unit is configured to utilize the calibration to convert the dark-field X-ray image or phase-contrast X-ray image of the region of interest of the object into an alveoli based image of the region of interest of the object. The image data in the converted image are represented in units of the alveoli index. The output unit is configured to output the alveoli based image.

In an example, the alveoli index is a number of alveoli.

According to an example, the calibration relates to how much dark-field signal or phase-contrast signal is associated with an alveolus.

According to an example, the calibration is a simulation generated calibration.

According to an example, the calibration was generated on the basis of a simulation of at least one sphere having a diameter equivalent to a diameter of a typical alveolus.

In case of a such a simulation either a wave propagation code or a Monte Carlo simulation can be used to determine the scattering angles of the x-rays when passing through the lung tissue. More details on a wave propagation code can be found in the paper by Wolf, Johannes, et al. "Fast one-dimensional wave-front propagation for x-ray differential phase-contrast imaging." Biomedical optics express 5.10 (2014): 3739-3747, and more details on a Monte Carlo simulation can be found in the paper by Peter, Silvia, et al. "Combining Monte Carlo methods with coherent wave optics for the simulation of phase-sensitive X-ray imaging." Journal of synchrotron radiation 21.3 (2014): 613-622. Thereby, the scattering angles are calculated based on the complex index of refraction and geometry of the object. The correct spectrum is achieved through using X-ray photons with different energies, so that the typical spectrum distribution is achieved. The gratings have to be taken into account during simulation, so that the scattering angles (and hence the position where the photons will reach the detector) can be translated into a dark-field signal for example. Since alveoli in the lung have approximately the shape of a sphere for the simulation, the lung can be assumed to consist of a number of spheres. Thereby, the radius of the spheres should be similar to the actual size of the alveoli in the lung. Another more sophisticated approach to obtain a lung sample with the correct geometry for the simulation, relies on a high resolution tomographic imaging of a lung sample, so that the exact alveoli structure can be directly resolved. Then the three-dimensional information can be used as an object for the simulation. The index of refraction for the alveoli tissue is a known tabulated value, while the inner content of the alveoli can be assumed to be air.

According to an example, the calibration is an experimentally generated calibration.

According to an example, the calibration was generated on the basis of a comparison between a calibration CT image of a lung and a calibration dark-field image or calibration phase-contrast image of the lung.

According to an example, generation of the calibration comprises determining a dark-field signal or phase-contrast signal at one or more positions of the calibration dark-field image or calibration phase-contrast image and determining a number of alveoli corresponding to the one or more positions of the calibration CT image.

According to an example, generation of the calibration comprises dividing the dark-field signal or phase-contrast signal at the one or more positions of the calibration dark-field image or calibration phase-contrast image by the number of alveoli corresponding to the one or more positions of the calibration CT image.

According to an example, the region of interest of the object comprises a lung.

According to an example, dark-field signal or phase-contrast signal outside of the lung in the dark-field or phase-contrast X-ray image of a region of interest of the object is set to a value that is outside of the range of values in the lung

According to an example, the value is zero.

Fig. 2 shows an example of a system 100 for alveoli based visualization in dark-field or phase-contrast X-ray imaging. The system 100 comprises at least one image acquisition unit 110, and an apparatus 10 for alveoli based visualization in dark-field or phase-contrast X-ray imaging as described above with respect to Fig. 1. The at least one image acquisition unit 110 is configured to provide the X-ray attenuation image, and to provide the dark-field or phase-contrast X-ray image.

In an example, the at least one image acquisition unit comprises a grating based phase-contrast and dark-field X-ray imaging device in an interferometer arrangement.

In an example, the at least one image acquisition unit comprises an X-ray imaging device. For example, the device can be a tomography arrangement, or a CT arrangement.

In an example, the same image acquisition unit can be used to acquire the attenuation and dark-field or phase-contrast images.

In an example, a phase-contrast image, and a dark-field image can be generated at the same time.

In an example, the interferometer arrangement comprises a Talbot interferometer. In an example, the interferometer arrangement comprises a phase grating configured to modulate onto the X-rays emitted by the source an interference pattern detectable by the X-ray detector as X-ray fringes. In an example, the interferometer arrangement comprises a second absorption grating configured to analyze the interference pattern. In an example, the second diffraction grating is an absorption grating. In an example, the two gratings are arranged on mutually opposite sides of an examination region. In an example, the two gratings are arranged on the same side of an examination region. In an example, the interferometer comprises a source grating in addition to the one or two gratings already discussed. In this example, the source grating is located relatively close to the X-ray source and serves to make the X-rays propagating after the source grating partly coherent. In other words, an X-ray source can be adapted so as to emit radiation that is more coherent than if the source grating was not present. Therefore, in some examples a source grating is not required, for example when the X-ray source already produces suitably coherent X-rays. In an example, the interferometer arrangement is configured to produce Moiré fringes. In an example, the interferometer arrangement is purposely detuned such that some fringes are present in a detector area. In an example, the interferometer arrangement is purposely detuned by having a first grating inclined at a small angle to a second grating. In an example, detuning leads to the generation of Moiré fringes on the detector.

In an example, the output unit outputs a phase-contrast (or differential phase) image. In an example, the output unit outputs a dark-field image. In an example, the output unit outputs any combination of alveoli based image, phase-contrast and dark-field images. In other words, the output unit can simultaneously output all three types of image. In an example, the output unit outputs data representative of the object on a monitor such as a visual display unit or on a number of separate monitors. For example, alveoli based image, phase-contrast and dark-field images can be presented on a single monitor or presented on separate monitors.

In an example, the system has useful application in a clinical environment such as a hospital. In an example, the system can be used for diagnostic radiology and interventional radiology for the medical examination of the lungs of patients.

In an example, the at least one image acquisition unit is configured to acquire the calibration CT image.

In an example, the at least one image acquisition unit is configured to acquire the calibration dark-field or calibration phase-contrast image.

In an example, an image acquisition unit, such as a C-arm system, is used to acquire the calibration CT image, and a different image acquisition unit, such as a DPCI system, is used to acquire the calibration dark-field and/or calibration phase-contrast image. Then, as part of determining the calibration the CT image and dark-field or phase-contrast image can be registered one with the other, and the CT image used to count the number of alveoli at a region of a lung and the dark-field or phase contrast-image can then be used to determine how much dark-field or phase-contrast signal is associated with individual alveoli. This calibration can be done once, for example for a healthy subject, and can then be applied to all subsequent dark-field or phase-contrast images acquired of patients.

Fig. 3 shows a method 200 for alveoli based visualization in dark-field or phase-contrast X-ray imaging in its basic steps. The method 200 comprises:
- in a providing step 210, also referred to as step a), providing from an input unit to a processing unit a dark-field or phase-contrast X-ray image of a region of interest of an object, wherein image data are represented in units of a dark-field or a phase-contrast signal;
- in a providing step 220, also referred to as step b), providing from the input unit to the processing unit a calibration relating dark-field or phase-contrast signal to an alveoli index in a lung;
- in a utilizing step 230, also referred to as step c), utilizing by the processing unit the calibration to convert the dark-field or phase-contrast X-ray image of the region of interest of the object into an alveoli based image of the region of interest of the object, wherein the image data are represented in units of the alveoli index; and
- in an outputting step 240, also referred to as step d), outputting by an output unit the alveoli based image.

In an example, the calibration relates to how much dark-field or phase-contrast signal is associated with an alveolus.

In an example, the calibration is a simulation generated calibration.

In an example, the simulation was generated on the basis of a simulation of at least one sphere having a diameter equivalent to a diameter of a typical alveolus.

In an example, the calibration is an experimentally generated calibration.

In an example, the calibration was generated on the basis of a comparison between a calibration CT image of a lung and a calibration dark-field or calibration phase-contrast image of the lung.

In an example, the generation of the calibration comprised determining a dark-field or phase-contrast signal at one or more positions of the calibration dark-field or calibration phase-contrast image and determining a number of alveoli corresponding to the one or more positions of the calibration CT image.

In an example, determining the calibration comprised dividing the dark-field or phase-contrast signal at the one or more positions of the calibration dark-field or calibration phase-contrast image by the number of alveoli corresponding to the one or more positions of the calibration CT image.

In an example, the region of interest of the object comprises a lung.

In an example, dark-field or phase-contrast signal outside of the lung in the dark-field or phase-contrast X-ray image of the region of interest of the object is set to a value that is outside of the range of values in the lung

In an example, the value is zero.

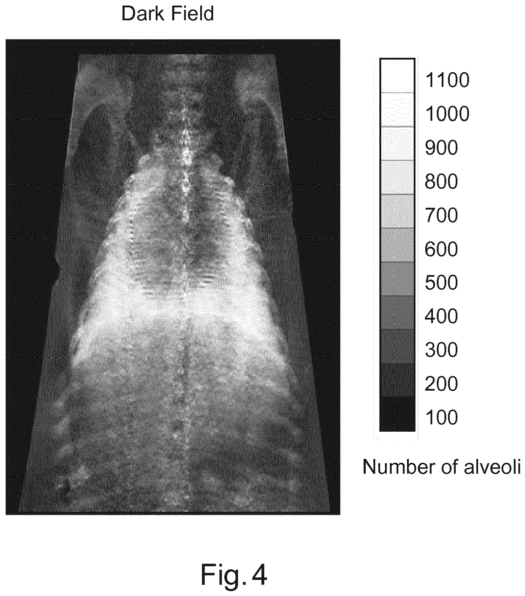

Fig. 4 shows an exemplary dark-field image of a porcine thorax, where the dark-field signal has been normalized to the number of alveoli. The colorbar, which is in black and white but could be in colour, shows the number of alveoli at different positions and this information can be easily and intuitively understood by a skilled clinician, who can immediately detect if there is an abnormality. The image in fig. 4 has been adapted from: Hellbach, Katharina, et al. "Depiction of pneumothoraces in a large animal model using x-ray dark-field radiography." Scientific reports 8.1 (2018): 2602.

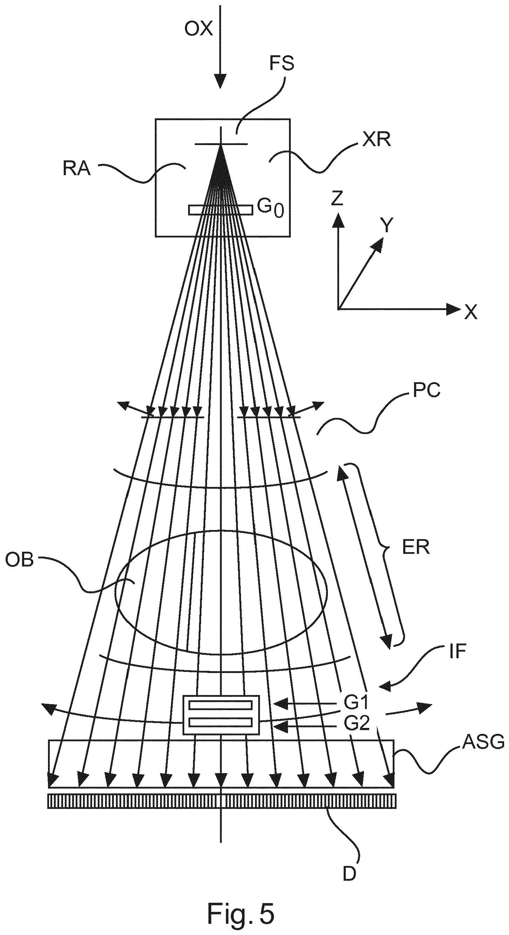

Fig. 5 shows an example of an X-ray phase contrast system that can also acquire dark-field images. The system can also acquire X-ray attenuation images. The system is capable of imaging for the spatial distribution of absorption of, or in, an object OB and also capable of imaging for the spatial distribution of refraction (phase-contrast imaging) and also capable of imaging for the spatial distribution of small angle scattering (dark-field imaging). The apparatus has a grating based interferometer IF that can be scanned across a stationary X-ray detector D, or if the gratings are big enough to cover the whole area of the detector no scanning is required. In this example, the interferometer IF comprises three grating structures G0, G1 and G2, although in other examples a two grating interferometer (having only a gratings G0 and G1 or Gland G2) is used.

In Fig. 5, the grating G1 is an absorption grating (but also can be a phase shift grating) whereas G2 is an absorption gating. The system further comprises an X-ray source XR and the X-ray detector D. The X-ray detector D can be a 2D full view X-ray detector, which is either planar or curved. A plurality of detector pixels are arranged in rows and columns as an array to form a 2D X-ray radiation sensitive surface capable of registering X-ray radiation emitted by the X-ray source.

The X-ray detector D and the X-ray source are spaced apart to form an examination region ER. The examination region is suitably spaced to receive the object OB to be imaged. The object is that of a patient's chest in order to examine the lung.

The interferometric grating structures G1 and G2 are arranged in the examination region ER between the X-ray source XR and X-ray detector D. The X-ray source XR has a focal spot FS from which the X-ray radiation beam emerges. It is the space between the focal spot FS and the X-ray detector's radiation sensitive surface where the two or three grating structures are arranged. The grating G1 is a phase grating and the grating G2 is an analyzer grating. In the example shown, there is in addition to the interferometric gratings G1, G2 of the interferometer IF, a further grating G0 which is the source grating.

The source grating G0 is arranged in proximity of the X-ray source XR, for example at the exit window of a housing of the X-ray tube. The function of the source grating G0 is to make the emitted radiation at least partly coherent. In other words, the source grating G0 can be dispensed with if an X-ray source is used which is capable of producing coherent radiation.

In operation the at least partly coherent radiation passes through the examination region ER and interacts with the object OB. The object then modulates attenuation, refraction, and small angle scattering information onto the radiation which can then be extracted by operation of the grating tandem G1 and G2. The gratings G1, G2 induce an interference pattern which can be detected at the X-ray detector D as fringes of a Moiré pattern. If there was no object in the examination region, there would still be an interference patter observable at the X-ray detector D, called the reference pattern which is normally captured during a calibration procedure. This comes about by especially adjusting or "detuning" the mutual spatial relationship between the two gratings G1 and G2 by inducing a slight flexure for instance so that the two gratings are not perfectly parallel. Now, if the object is positioned in the examination region and interacts with the radiation as mentioned, the Moiré pattern, which is now more appropriately called the object pattern, can be understood as a disturbed version of the reference pattern. This difference from the reference pattern can then be used to compute one or all of the phase-contrast, dark-field images.

Continuing with Fig. 5, to be able to acquire suitable signals from which the images can be computed, a scanning motion is performed by the grating tandem G1-G2, which as discussed above is not necessary if the gratings are big enough. As a result of this, at each pixel of the X-ray detector D a series of intensity values are detected. For good results, the detuning of the gratings G1, G2 is such that a period of the Moiré pattern should extend for a few of its cycles (two or three) in the direction of the scan motion. For each X-ray detector pixel, the series of intensity values can then be fitted to a (sinusoidal) signal forward model, for example, in order to derive the respective contributions of refraction, absorption, and small angle scatter. This type of signal processing is done in a signal processing unit not shown in Fig. 5, but which is known to the skilled person. The X-ray detector D remains stationary for any given orientation of the optical axis OX which is shown in Fig. 5 to extend along the Z axis. In other words, the X-ray detector D is kept stationary (at least during an image acquisition operation) with respect to an arbitrary reference point in the examination region. The interferometric setup as described above is what is commonly referred to as a Talbot-Lau interferometer. The distances between G0 and G1 and between G1 and G2 must be finely tuned to fit the requirements of Talbot distance which in turn is a function of the "pitch" (that is, the spatial period of the grating rulings) of the respective grating. Moving the interferometer IF relative to the X-ray detector D may cause a slight change in fringe distribution due to fringe drift. However, the fringe drift can be compensated by relating such drift to the fringe drift as obtained with a reference scan. Such reference scan may be a blank scan performed at the installation of the X-ray imaging apparatus.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

## Claims

1. An apparatus (10) for alveoli based visualization in dark-field or phase-contrast X-ray imaging, comprising:
- an input unit (20);
- a processing unit (30); and
- an output unit (40);
wherein, the input unit is configured to provide the processing unit with a dark-field or phase-contrast X-ray image of a region of interest of an object, wherein image data are represented in units of a dark-field signal or a phase-contrast signal;
**characterised in that**
the input unit is configured to provide the processing unit with a calibration relating dark-field signal or phase-contrast signal to a number of alveoli in a lung; wherein the calibration is an experimentally generated calibration that relates to how much dark-field signal or phase-contrast signal is associated with an alveolus, wherein the calibration was generated on the basis of a comparison between a high resolution calibration CT image of a calibration lung and a calibration dark-field image or calibration phase-contrast image of the calibration lung, and wherein the calibration CT image had a resolution high enough to reveal a number of alveoli in the calibration lung;
wherein, the processing unit is configured to utilize the calibration to convert the dark-field X-ray image or phase-contrast X-ray image of the region of interest of the object into an alveoli based image of the region of interest of the object, wherein the image data are represented in units of the number of alveoli; and
wherein, the output unit is configured to output the alveoli based image.

2. Apparatus according to claim 1, wherein the generation of the calibration comprises determining a dark-field signal or phase-contrast signal at one or more positions of the calibration dark-field image or calibration phase-contrast image and determining a number of alveoli corresponding to the one or more positions of the calibration CT image.

3. Apparatus according to claim 2, wherein the generation of the calibration comprises dividing the dark-field signal or phase-contrast signal at the one or more positions of the calibration dark-field image or calibration phase-contrast image by the number of alveoli corresponding to the one or more positions of the calibration CT image.

4. Apparatus according to any of claims 1-8, wherein the region of interest of the object comprises a lung.

5. Apparatus according claim 4, wherein dark-field signal or phase-contrast signal outside of the lung in the dark field or phase contrast X-ray image of a region of interest of the object is set to a value that is outside of the range of values in the lung

6. Apparatus according to claim 5, wherein the value is zero.

7. A system (100) for alveoli based visualization in dark-field or phase-contrast X-ray imaging, the system comprising:
- at least one image acquisition unit (110); and
- an apparatus (10) for alveoli based visualization in dark-field or phase-contrast X-ray imaging according to any of the preceding claims; and
wherein, the at least one image acquisition unit is configured to provide the X-ray attenuation image, and to provide the dark-field or phase-contrast X-ray image.

8. A method (200) for alveoli based visualization in dark-field or phase-contrast X-ray imaging, comprising:
a) providing (210) a dark-field or phase-contrast X-ray image of a region of interest of an object, wherein image data are represented in units of a dark-field or a phase-contrast signal; **characterised by**
b) providing (220) a calibration relating dark-field or phase-contrast signal to a number of alveoli in a lung; wherein the calibration is an experimentally generated calibration that relates to how much dark-field signal or phase-contrast signal is associated with an alveolus, and wherein the calibration was generated on the basis of a comparison between a high resolution calibration CT image of a calibration lung and a calibration dark-field image or calibration phase-contrast image of the calibration lung, wherein the calibration CT image had a resolution high enough to reveal a number of alveoli in the calibration lung;
c) utilizing (230) the calibration to convert the dark-field or phase-contrast X-ray image of the region of interest of the object into an alveoli based image of the region of interest of the object, wherein the image data are represented in units of the number of alveoli; and
d) outputting (240) the alveoli based image.

9. A computer program element for controlling an apparatus according to one of claims 1 to 6 and/or system of claim 7, which when executed by a processor is configured to carry out the method of claim 8.

10. A computer readable medium having stored the program element of claim 9.

## Patentansprüche

1. Ein Apparat (10) für die alveolenbasierte Visualisierung in Dunkelfeld- oder Phasen- Kontrast-Röntgenbildgebung, umfassend:
- eine Eingabeeinheit (20);
- eine Verarbeitungseinheit (30); und
- eine Ausgabeeinheit (40);
wobei die Eingabeeinheit konfiguriert ist, um der Verarbeitungseinheit ein Dunkelfeld- oder Phasenkontrast-Röntgenbild einer Region von Interesse oder eines Objektes bereitzustellen, wobei die Bilddaten in Einheiten eines Dunkelfeldsignals oder eines Phasenkontrastsignals dargestellt werden; **dadurch gekennzeichnet, dass** die Eingabeeinheit konfiguriert ist, der Verarbeitungseinheit eine Kalibrierung, die ein Dunkelfeldsignal oder ein Phasenkontrastsignal mit einer Anzahl von Alveolen in einer Lunge in Beziehung setzt, bereitzustellen; wobei die Kalibrierung eine experimentell erzeugte Kalibrierung ist, die sich darauf bezieht, wie viel Dunkelfeldsignal oder Phasenkontrastsignal einer Alveole zugeordnet ist, wobei die Kalibrierung auf der Grundlage eines Vergleichs zwischen einem hochauflösenden Kalibrierungs-CT-Bild einer Kalibrierlunge und einem Kalibrier-Dunkelfeldbild oder einem Kalibrier-Phasenkontrastbild der Kalibrierlunge erzeugt wurde, und wobei das Kalibrier-CT-Bild eine Auflösung hatte, die hoch genug war, um eine Anzahl von Alveolen in der Kalibrierlunge sichtbar zu machen; wobei die Verarbeitungseinheit konfiguriert ist, die Kalibrierung zu verwenden, um das Dunkelfeld-Röntgenbild oder das Phasenkontrast-Röntgenbild des interessanten Bereichs des Objekts in ein alveolenbasiertes Bild des interessanten Bereichs des Objekts umzuwandeln, wobei die Bilddaten in Einheiten der Anzahl der Alveolen dargestellt sind; und
wobei die Ausgabeeinheit konfiguriert ist, als Ausgabe das alveolenbasierte Bild zu liefern.

2. Apparat entsprechend Anspruch 1, wobei die Erzeugung der Kalibrierung es umfasst, ein Dunkelfeldsignal oder Phasenkontrastsignal bei einer oder mehreren Positionen des Kalibrierungs-Dunkelfeldbildes oder des Kalibrierungs-Phasenkontrastbildes zu bestimmen und eine Anzahl von Alveolen zu bestimmen, welche der einen oder den mehreren Positionen des Kalibrierungs-CT-Bildes entsprechen.

3. Gerät entsprechend Anspruch 2, wobei die Erzeugung der Kalibrierung es umfasst, dass das Dunkelfeldsignal oder das Phasenkontrastsignal bei der einen oder den mehreren Positionen des Kalibrierungs-Dunkelfeldbildes oder des Kalibrierungs-Phasenkontrastbildes durch die Anzahl von Alveolen geteilt wird, welche der einen oder den mehreren Positionen des Kalibrierungs-CT-Bildes entspricht.

4. Apparat entsprechend einem der Ansprüche 1-8, wobei der interessante Bereich des Objektes eine Lunge umfasst.

5. Apparat entsprechend Anspruch 4, wobei das Dunkelfeldsignal oder das Phasenkontrast- Signal außerhalb der Lunge im Dunkelfeld- oder Phasenkontrast-Röntgenbild eines interessanten Bereiches des Objekts auf einen Wert außerhalb der Bandbreite der Werte in der Lunge eingestellt ist.

6. Apparat entsprechend Anspruch 5, wobei der Wert Null beträgt.

7. Ein System (100) für die alveolenbasierte Visualisierung in Dunkelfeld- oder Phasenkontrast-Röntgenbildgebung, das System umfasst:
- Wenigstens eine Bilderfassungseinheit (110); und
- einen Apparat (10) für die alveolenbasierte Visualisierung in Dunkelfeld- oder Phasenkontrast-Röntgenbildgebung gemäß einem der vorangegangenen Ansprüche; und wobei die wenigstens eine Bilderfassungseinheit konfiguriert ist, das Röntgen-Dämpfungsbild bereitzustellen, und das Dunkelfeldoder Phasenkontrast-Röntgenbild bereitzustellen.

8. Eine Methode (200) für die alveolenbasierte Visualisierung in Dunkelfeld- oder Phasenkontrast-Röntgenbildgebung, umfassend:
a) Bereitstellung (210) eines Dunkelfeld- oder Phasenkontrast-Röntgenbildes eines interessanten Bereichs eines Objektes, wobei Bilddaten in Einheiten eines Dunkelfeld- oder Phasenkontrastsignals dargestellt sind; **gekennzeichnet durch**
b) Bereitstellung (220) eines auf Kalibrierung bezogenen Dunkelfeld- oder Phasenkontrastsignals zu einer Anzahl von Alveolen in einer Lunge; wobei die Kalibrierung eine experimentell erzeugte Kalibrierung ist, die sich darauf bezieht, wie viel Dunkelfeldsignal oder Phasenkontrastsignal einer Alveole zugeordnet ist, wobei die Kalibrierung auf der Grundlage eines Vergleichs zwischen einem hochauflösenden Kalibrierungs-CT-Bild einer Kalibrierlunge und einem Kalibrier-Dunkelfeldbild oder einem Kalibrier-Phasenkontrastbild der Kalibrierlunge erzeugt wurde, und wobei das Kalibrier-CT-Bild eine Auflösung hatte, die hoch genug war, um eine Anzahl von Alveolen in der Kalibrierlunge sichtbar zu machen;
c) Verwendung (230) der Kalibrierung, das Dunkelfeld- oder Phasenkontrast-Röntgenbild des interessanten Bereichs des Objekts in ein alveolenbasiertes Bild des interessanten Bereichs des Objekts umzuwandeln, wobei die Bilddaten in Einheiten der Anzahl der Alveolen dargestellt sind; und
d) Ausgabe (240) des alveolenbasierten Bildes.

9. Ein Computerprogramm zur Steuerung eines Apparats gemäß einem der Ansprüche 1 bis 6 und/oder dem System von Anspruch 7, welches, wenn es durch einen Prozessor ausgeführt wird, konfiguriert ist, die Methode von Anspruch 8 durchzuführen.

10. Ein durch Computer lesbares Medium, welches das Programmelement von Anspruch 9 gespeichert hat.

## Revendications

1. Un appareil (10) pour une visualisation à partir des alvéoles en champ sombre ou à contraste de phase en imagerie à rayons X, comprend:
- une unité d'entrée (20);
- une unité de traitement (30); et
- une unité de sortie (40);
où, l'unité d'entrée est configurée pour fournir à l'unité de traitement avec une imagerie à rayons X en champ sombre ou à contraste de phase une zone d'intérêt d'un objet, où les données d'imagerie sont représentées dans des unités d'un signal en champ sombre ou un signal à contraste de phase;
**caractérisé par le fait que** l'unité d'entrée est configurée pour fournir à l'unité de traitement un calibrage relatif au signal en champ sombre ou au signal à contraste de phase à un certain nombre d'alvéoles dans le poumon; où le calibrage est un calibrage généré de manière expérimentale qui se rapporte à la quantité de signaux de champ sombre ou de signal à contraste de phase associé à une alvéole, où le calibrage était généré à partir de la comparaison entre une image CT de calibrage à haute résolution d'un calibrage de poumon et un calibrage d'une image en champ sombre ou un calibrage d'une image à contraste de phase du calibrage du poumon, et où le calibrage d'une image CT avait une résolution assez élevée pour révéler un certain nombre d'alvéoles dans le calibrage du poumon;
où, l'unité de traitement est configurée pour utiliser le calibrage pour convertir l'imagerie à rayons X en champ sombre ou l'imagerie à rayons X à contraste de phase de la zone d'intérêt de l'objet en une imagerie fondée à partir d'alvéoles de la zone d'intérêt de l'objet; où les données d'imagerie sont représentées en unités du nombre d'alvéoles; et où, l'unité de sortie est configurée pour produire l'image à partir des alvéoles.

2. Un appareil selon la revendication 1, où la production d'un calibrage comprend la détermination d'un signal en champ sombre ou d'un signal à contraste de phase en une ou plusieurs positions d'un calibrage d'une image en champ sombre ou d'un calibrage d'une image à contraste de phase et la détermination d'un certain nombre d'alvéoles correspondant à une ou plusieurs positions d'un calibrage d'une image CT.

3. Un appareil selon la revendication 2, où la production d'un calibrage comprend la division d'un signal en champ sombre ou d'un signal à contraste de phase en une ou plusieurs positions d'un calibrage d'une image en champ sombre ou d'un calibrage d'une image à contraste de phase par le nombre d'alvéoles correspondant à une ou plusieurs positions d'un calibrage d'une image CT.

4. Un appareil selon l'une des revendications 1-8, où la zone d'intérêt de l'objet comprend un poumon.

5. Un appareil selon la revendication 4, où le signal en champ sombre ou le signal à contraste de phase en dehors du poumon dans le champ sombre ou l'imagerie à rayons X à contraste de phase d'une zone d'intérêt de l'objet est réglée pour une valeur se trouvant en dehors de la plage de valeurs du poumon.

6. Un appareil selon la revendication 5, où la valeur est zéro.

7. Un appareil (100) pour une visualisation à partir des alvéoles en imagerie à rayons X en champ sombre ou à contraste de phase, le système comprend:
- au moins une unité d'acquisition d'image (110); et
- un appareil (10) pour une visualisation à partir des alvéoles en imagerie à rayons X à contraste de phase ou en champ sombre selon l'une des quelconques des revendications précédentes;
où, au moins une unité d'acquisition d'image est configurée pour fournir l'image d'atténuation des rayons X, et pour fournir l'imagerie à rayons X en champ sombre ou à contraste de phase.

8. Une méthode (200) pour une visualisation à partir des alvéoles en imagerie à rayons X en champ sombre ou à contraste de phase, comprenant:
a) la fourniture (210) d'une imagerie à rayons X en champ sombre ou à contraste de phase d'une zone d'intérêt de l'objet, où les données d'imagerie sont représentées dans des unités d'un signal en champ sombre ou un signal à contraste de phase; **caractérisés par**
b) la fourniture (220) d'un calibrage relatif au signal en champ sombre ou au signal à contraste de phase à un certain nombre d'alvéoles dans le poumon; où le calibrage est un calibrage généré de manière expérimentale qui se rapporte à la quantité de signaux de champ sombre ou de signal à contraste de phase associé à une alvéole, où le calibrage était générée à partir de la comparaison entre une image CT de calibrage à haute résolution d'un calibrage de poumon et un calibrage d'une image en champ sombre ou un calibrage d'une image à contraste de phase du calibrage du poumon, et où le calibrage d'une image CT avait une résolution assez élevée pour révéler un certain nombre d'alvéoles dans le calibrage du poumon;
c) l'utilisation (230) du calibrage pour convertir l'imagerie à rayons X en champ sombre ou l'imagerie à rayons X à contraste de phase de la zone d'intérêt de l'objet en une imagerie fondée à partir d'alvéoles de la zone d'intérêt de l'objet; où les données d'imagerie sont représentées en unités du nombre d'alvéoles; et
d) la sortie (240) de l'imagerie alvéolaire.

9. Un élément de programme informatique pour contrôler un appareil selon l'une des revendications 1 à 6 et/ou le système de la revendication 7, qui lorsqu'il est exécuté par un processeur est configuré pour mettre en œuvre la méthode de la revendication 8.

10. Un support lisible par ordinateur a stocké un élément de programme de la revendication 9.
